(19) Europäisches Patentamt
European Patent Office
Office européen des brevets

(11) EP 1 592 408 B1

(12) **EUROPEAN PATENT SPECIFICATION**

(45) Date of publication and mention of the grant of the patent:
**26.08.2009 Bulletin 2009/35**

(21) Application number: **04704837.6**

(22) Date of filing: **23.01.2004**

(51) Int Cl.:
***A61K 9/22*** *(2006.01)*

(86) International application number:
**PCT/US2004/001718**

(87) International publication number:
**WO 2004/066979 (12.08.2004 Gazette 2004/33)**

(54) **SUSTAINED RELEASE DEVICE AND METHOD FOR OCULAR DELIVERY OF ADRENERGIC AGENTS**

VORRICHTUNG MIT VERLÄNGERTER FREISETZUNG UND VERFAHREN ZUR OKULAREN VERABREICHUNG VON ADRENERGEN WIRKSTOFFEN

DISPOSITIF ET PROCEDE A LIBERATION PROLONGEE POUR L'ADMINISTRATION OCULAIRE D'AGENTS ADRENERGIQUES

(84) Designated Contracting States:
**AT BE BG CH CY CZ DE DK EE ES FI FR GB GR HU IE IT LI LU MC NL PT RO SE SI SK TR**

(30) Priority: **24.01.2003 US 442499 P**
**26.06.2003 US 483316 P**
**26.06.2003 US 482677 P**
**11.09.2003 US 501974 P**

(43) Date of publication of application:
**09.11.2005 Bulletin 2005/45**

(73) Proprietor: **PSivida US Inc.**
**Watertown MA 02472 (US)**

(72) Inventors:
- **ASHTON, Paul**
  **Boston, MA 02108-1025 (US)**
- **GUO, Hong**
  **Belmont, MA 02478 (US)**

(74) Representative: **Vossius & Partner**
**Siebertstrasse 4**
**81675 München (DE)**

(56) References cited:
**US-A- 5 378 475**
**US-A- 5 902 598**
**US-B1- 6 196 993**
**US-B1- 6 375 972**

EP 1 592 408 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### Field of the Invention

The present invention relates to the field of sustained drug delivery to the eye, and particularly to the treatment and/or prevention of raised intraocular pressure, such as that associated with glaucoma or the use of corticosteroids, by sustained delivery of adrenergic agents to the eye.

### Background of the Invention

1. Adrenergic Agents.

Glaucoma is one of the leading causes of blindness in the developed countries of the world. The chief pathophysiological feature of glaucoma is raised intraocular pressure. Surgery and/or drugs intended to lower intraocular pressure are the most common treatments for glaucoma. Among the principal pharmaceutical treatments in use today are the administration of miotics (*e.g.*, pilocarpine, carbachol and echothiophate), which open the trabecular meshwork to increase the rate of fluid flow out of the eye; PGF-2$\alpha$ analogues (*e.g.*, unoprostone, travoprost, bimatoprost and latanoprost), which enhance uveoscleral outflow; and carbonic anhydrase inhibitors (*e.g.*, acetazolamide, methazolamide, and dorzolamide), which decrease the rate of fluid flow into the eye.

Adrenergic agents have also proven useful in treating elevated intraocular pressure. Both $\beta$-adrenergic antagonists and $\alpha_1$- and $\alpha_2$-adrenoceptor agonists are prescribed for individuals suffering from glaucoma, and also to control or prevent the elevations in intraocular pressure that frequently occur after ocular laser surgery. Typical $\alpha_2$-agonists (e.g., dipivefrin, brimonidine) reduce the tone of the sympathetic system at the ciliary process level, which leads to a decrease of aqueous humor synthesis. Another $\alpha$-adrenoceptor agonist, apraclonidine, reportedly exhibits both $\alpha_1$, and $\alpha_2$-adrenoceptor activity, and at least one $\alpha_1$-adrenoceptor agonist (bunazosin) has been developed for the treatment of elevated intraocular pressure.

Beta-adrenergic antagonists stimulate ciliary adenylyl cyclase activity. Examples of $\beta$-adrenergic antagonists that are effective in reducing intraocular pressure include timolol, betaxolol, levobetaxolol, levobunalol, carteolol, isoprenaline, fenoterol, metipranolol and clenbuterol. Both selective ($\beta_2$) and non-selective ($\beta_1$ and $\beta_2$) antagonists have been developed.

Most of the topical adrenergic agents are relatively short-term agents that must be administered two or three times daily. Furthermore, self-administration of eye drops often results in a substantial portion of the drop being lost due to overflow. A substantial portion of the drug solution that is delivered to the ocular surface is then immediately washed away by tears, and that portion of the drug which does penetrate the cornea results in an initial peak tissue concentration, followed by a gradual decrease, so that before the next administration of the eye drops the tissue concentration may be below the concentration needed to create the intended pharmacological effect. The variable and intermittent topical application of eye drops, combined with the vagaries of patient compliance with the prescribed regimen, result in cycles of high and low concentrations of topical anti-glaucoma agents in the eye, and the possible cycling of intraocular pressure. Because the damage to the optic nerve caused by raised intraocular pressure can be cumulative, the ideal treatment would maintain a therapeutically effective amount of drug in the eye at all times.

Topical beta-adrenergic blocking agents are absorbed systemically, and in patients with severe impairment of myocardial function, they may inhibit the sympathetic stimulatory effect necessary to maintain adequate cardiac output. Furthermore, beta-adrenergic receptor blockade in the bronchi and bronchioles may result in significantly increased airway resistance from unopposed parasympathetic activity. Such an effect is potentially dangerous in patients with asthma or other bronchospastic conditions. Cases of death due to bronchospasm in patients with asthma, and death in association with cardiac failure, have been reported in connection with the use of topical adrenergic agents.

2. Ocular Drug Delivery Devices

Certain sustained-release devices and formulations adapted for administration of drugs to the eye have been described previously in the art. U.S. Patent No. 6,196,993 issued to Cohan and Diamond describes an ophthalmic insert intended for implantation into the lacrimal canaliculus of the eye. This device contains an internal reservoir of drug, and features a surface opening through which the drug is intended to diffuse. Sustained-release systems adapted for placement between the lower lid and the eye are disclosed in U.S. Patent Nos. 3,416,530 and 3,618,604 issued to Ness, 3,626,940 issued to Zaffaroni, 3,826,258 issued to Abraham, 3,845,201 issued to Haddad and Loucas, 3,845,770 issued to Theeuwes et al., 3,962,414 issued to Michaels, 3,993,071 issued to Higuchi et al., 4,014,335 issued to Arnold, and 4,164,559 issued to Miyata. U.S. Patent No. 5,824,072 issued to Wong describes reservoir and polymer matrix ocular implants intended for implantation, for example, into the choroid. U.S. Patent No. 5,476,511 issued to Gwon et al.

describes an ocular implant intended for implantation beneath the conjunctiva. U.S. Patent No. 6,416,777 issued to Yaacobi describes an ocular implant for implantation onto the outer surface of the sclera at the back of the eye.

Devices such as those described above typically consist of a drug-containing reservoir surrounded by a perforate or permeable membrane that controls the diffusion of the drug, or else a drug dispersed in a polymer matrix.

U.S. Patent No. 6,027,745 issued to Nakada describes a contact lens formed with an internal reservoir for containing and releasing drug substances, and U.S. Patent No. 6,368,615 issued to Guttag describes a contact lens having a releasable drug covalently bound to the lens material.

U.S. Patent Nos. 6,217,895 and 6,548,078 issued to Guo and Ashton describes the implantation of a sustained-release device into the vitreous cavity for release of a corticosteroid. U.S. Patent No. 5,378,475 issued to Smith et al. and U.S. Patent No. 5,902,598 issued to Chen and Ashton describe a sustained-release device comprising a drug core with two or more polymeric coatings, one of which is an impermeable layer that partially coats the core and controls drug release. The device is stated to be suitable for treating ocular conditions when implanted in the vitreous cavity. U.S. Patent Nos. 5,773,019 and 6,001,386 issued to Ashton and Pearson describe a device which is suitable for implantation into the vitreous cavity, having a core of a low-solubility drug and a single permeable coating. U.S. Patent No. 6,375,972, issued to Guo and Ashton describes a device comprising an inner core or reservoir including a drug, an inner tube impermeable to the passage of the drug, an impermeable member positioned at the first end of the tube, and a permeable member positioned at the second end of the tube through which the drug diffuses. Another embodiment of the '972 Patent includes an impermeable outer layer including a diffusion port that surrounds the inner tube, impermeable member, and permeable member.

A device intended to provide sustained release of a drug should also provide controlled release, i.e., it should approximate zero-order or linear release over time, so as to maintain not only prolonged release but also a relatively constant and therapeutically effective concentration of the drug. The duration of release should be sufficiently long so that the insertion of the device (and in the case of non-bioerodable devices, removal of expended devices) is not inconveniently frequent. This is particularly an issue where insertion and removal must be carried out by a medical professional. Depending on the condition to be treated, such devices may provide for controlled release over a period of weeks, months or even years.

In matrix systems, drug is dispersed throughout a polymeric matrix and is released as it dissolves and diffuses out of the matrix. In matrix devices, the drug dispersed in the matrix may be present either in dissolved or dispersed form. Release follows Fickian kinetics from devices where the drug is dissolved. When the drug is dispersed in the matrix, it is released according to $t^{1/2}$ kinetics until the concentration in the matrix falls below the saturation value, at which point the release rate slows down and Fickian release is observed. For these reasons, zero-order release can be difficult to achieve with matrix systems.

In some bioerodable systems, diffusion through the matrix is extremely slow, and drugs are intended to be released only as the matrix is degraded. It has proven to be difficult to use this approach to achieve zero-order release, because monolithic polymer devices do not ordinarily undergo zero-order degradation, and "S" type kinetics are more commonly observed.

An approximation of linear release is achievable when a drug reservoir is coated with a rate-controlling permeable membrane. Drug diffusion across the membrane is rate-limiting and is constant (zero order) as long as the membrane permeability and the solution concentration of drug in the reservoir remain constant (e.g., as long as there is undissolved drug in the reservoir).

Despite a great deal of effort in this field, the devices produced to date are not ideal in terms of meeting the requirements of zero-order release over time, prolonged release, and a relatively constant and therapeutically effective concentration of drug, while at the same time being acceptable to patients and medical professionals. In particular, there exists a need for an improved method for treating and/or preventing glaucoma and other indications associated with raised intraocular pressure by administering adrenergic agents to the eye in a manner that avoids the problems of variable drug concentration associated with topical administration without causing systemic side effects.

## Brief Description of the Invention

The present invention provides a device and its use for the manufacture of a medicament for treating and/or preventing raised intraocular pressure, such as that associated with glaucoma or the use of corticosteroids, with adrenergic agents, without the variability in local concentration associated with topically applied agents, and without the adverse side effects associated with systemic agents. The invention provides insertable sustained-release devices adapted to maintain a therapeutically effective concentration of one or more adrenergic agents within the ciliary body for an extended period of time.

Also described is a method for local application of one or more adrenergic agents to the eye, by means of the devices of the invention, and methods of treating intraocular pressure by ocular administration of one or more adrenergic agents, via insertion of the devices of the invention.

**Brief Description of the Drawings**

FIG. 1 is an enlarged cross-sectional illustration of one embodiment of a sustained release drug delivery device in accordance with the present invention.

FIG. 2 is an enlarged cross-sectional illustration of a second embodiment of a sustained release drug delivery device in accordance with the present invention.

FIG. 3 is an enlarged cross-sectional illustration of a third embodiment of a sustained release drug delivery device in accordance with the present invention.

FIG. 4 is a cross-sectional illustration of the embodiment illustrated in FIG. 2, taken at line 4-4.

FIG. 5 is a cross-sectional illustration of a sustained release drug delivery device in accordance with the present invention, adapted for insertion into a lacrimal duct.

**Detailed Description of the Invention**

The present invention provides a device and its use for the preparation of a medicament for delivering and maintaining a therapeutic amount of at least one adrenergic agent in the ciliary body of the eye of a patient for an extended period of time. The device is a sustained-release drug delivery device comprising at least one adrenergic agents, which can maintain a therapeutically effective concentration of the adrenergic agent (s) within the ciliary body for an extended period of time. Described is a method involving inserting such device into or in proximity to the eye of a patient, in order to deliver the adrenergic agent (s) to the ciliary body.

The device of the present invention may be adapted for insertion between the eye and eyelid, preferably the lower eyelid. It may, in alternative embodiments, be adapted for insertion into the anterior or posterior chambers, under the retina, into the choroid, or into or onto the sclera. In another embodiment, the device may be adapted for insertion into the lacrimal canaliculus. In yet another embodiment, the device may be a contact lens or intraocular lens, or it may be incorporated into or attached to a contact lens or intraocular lens.

As used herein, even if not particularly called out, the term "insert" means insert, inject, implant, or administer in any other fashion. The term "inserted" means inserted, injected, implanted, or administered in any other fashion. The term "insertion" means insertion, injection, implantation, or administration in any other fashion. Similarly, the term "insertable" means insertable, injectable, implantable, or otherwise adminstrable.

The term "patient," as used herein, refers to either a human or a non-human animal.

Codrugs or prodrugs may be used to deliver adrenergic agents in a sustained manner, and may be adapted for use in the embodiments of the present invention discussed herein. The term "codrug" as used herein means a compound comprising a first molecule residue associated with a second molecule residue, wherein each residue, in its separate form (e.g., in the absence of the association), is an active agent or a prodrug of an active agent. The association between said residues can be either ionic or covalent and, in the case of covalent associations, either direct or indirect through a linker. The first molecule can be the same or different from the second. Codrugs, as that term is used herein, are more fully described in U.S. Patent No. 6,051,576.

As used herein, the terms "drug," "agent," or "adrenergic agent" includes a codrug, prodrug, or a pharmaceutically acceptable salt form thereof. Pharmaceutically acceptable salts include, but are not limited to, sulfates, hydrochlorides, and the like where the compound is basic, and sodium salts where the compound is acidic.

As used herein, "sustained-release device" or "sustained-release formulation" means a device or formulation that releases an agent over an extended period of time in a controlled fashion. As also discussed elsewhere herein, examples of sustained-release devices and formulations suitable for the present invention may be found in U.S. Patent No. 6,375,972, U.S. Patent No. 5,378,475, U.S. Patent No. 5,773,019, and U.S. Patent No. 5,902,598.

In one embodiment, the present invention provides a sustained-release drug delivery device adapted for insertion into or adjacent to the eye of a patient, where the drug delivery device, in whole or in part, is formed by co-extruding (a) an inner drug-containing core comprising at least one adrenergic agent and (b) an outer polymeric layer. The outer layer, which preferably is tubular in shape, may be permeable, semi-permeable, or impermeable to the drug. The drug-containing core is formed by admixing the drug with a polymer matrix prior to formation of the device. The polymer matrix may or may not significantly affect the release rate of the drug. The outer layer, the polymer admixed with the drug-containing core, or both may be bioerodible. The co-extruded product can be segmented into a plurality of drug delivery devices. The devices may be left uncoated so that their respective ends are open, or the devices may

be coated with, for example, an additional polymeric layer that is permeable, semi-permeable, or impermeable to the drug.

As more fully described in U.S. Provisional Patent Application 10/428,214 and in U.S. Provisional Patent Application by Guo et al., filed September 11, 2003, entitled "Bioerodible Sustained Release Drug Delivery Systems", the co-extruded embodiment discussed above may be fabricated by forwarding a polymeric material to a first extrusion device, forwarding at least one drug to a second extrusion device, co-extruding a mass including the polymeric material and the drug, and forming the mass into at least one co-extruded drug delivery device that comprises a core including the drug(s) and an outer layer including the polymeric material. In certain embodiments, the drug(s) forwarded to the second extrusion device is in admixture with at least one polymer. The at least one polymer may be a bioerodible polymer, such as poly(vinyl acetate) (PVAC), polycaprolactone (PCL), polyethylene glycol (PEG), or poly(dl-lactide-co-glycolide) (PLGA). In certain embodiments, the drug(s) and the at least one polymer are admixed in powder form.

The outer layer may be impermeable, semi-permeable, or permeable to the drug disposed within the inner drug-containing core, and may comprise any biocompatible polymer, such as PCL, an ethylene/vinyl acetate copolymer (EVA), polyalkyl cyanoacralate, polyurethane, a nylon, or PLGA, or a copolymer of any of these. In certain embodiments, the outer layer is radiation curable. In certain embodiments, the outer layer comprises at least one drug, which may be the same or different than the drug used in the inner core.

While co-extrusion may be used to form a device according to the invention, other techniques may readily be used. For example, the core can be poured into a preformed tube otherwise having one or more of the characteristics of the present invention.

In certain embodiments, the drug delivery device (formed by any of the possible techniques) is in a tubular form, and may be segmented into a plurality of shorter products. In certain embodiments, the plurality of shorter products may be coated with one or more additional layers, including at least one of a layer that is permeable to the adrenergic agent(s), a layer that is semi-permeable to such drug(s), and a layer that is bioerodible. The additional layer(s) may include any biocompatible polymer, such as PCL, EVA, polyalkyl cyanoacralate, polyurethane, a nylon, or PLGA, or a copolymer of any of these.

Materials suitable to form the outer layer and inner drug-containing core, respectively, are numerous. In this regard, U.S. Patent 6,375,972 describes suitable materials for forming insertable co-extruded drug delivery devices, which materials are included among those usable as materials for the outer layer and inner drug-containing core. Preferably, the materials for certain embodiments of the present invention are selected for their ability to be extruded without negatively affecting the properties for which they are specified. For example, for those materials that are to be impermeable to the drug, a material is selected that, upon being processed through an extrusion device, is or remains impermeable. Similarly, biocompatible materials are preferably chosen for the materials that will, when the drug delivery device is fully constructed, come in contact with the patient's biological tissues. Suitable materials include PCL, EVA, PEG, poly(vinyl acetate) (PVA), poly(lactic acid) (PLA), poly(glycolic acid) (PGA), PLGA, polyalkyl cyanoacralate, polyurethane, nylons, or copolymers thereof In polymers including lactic acid monomers, the lactic acid may be D-, L-, or any mixture of D- and L- isomers.

The selection of the material(s) to form the inner drug-containing core involves additional considerations. As one of skill in the art readily appreciates, extrusion devices typically include one or more heaters and one or more screw drives, plungers, or other pressure-generating devices; indeed, it may be a goal of the extruder to raise the temperature, fluid pressure, or both, of the material being extruded. This can present difficulties when a pharmaceutically active drug included in the materials being processed and extruded by the extruder is heated and/or exposed to elevated pressures. This difficulty can be compounded when the drug itself is to be held in a polymer matrix, and therefore a polymer material is also mixed and heated and/or pressurized with the drug in the extruder. The materials may be selected so that the activity of the drug in the inner drug-containing core is sufficient for producing the desired effect when inserted in a patient. Furthermore, when the drug is admixed with a polymer for forming a matrix upon extrusion, the polymer material that forms the matrix is advantageously selected so that the drug is not destabilized by the matrix. Preferably, the matrix material is selected so that diffusion through the matrix has little or no effect on the release rate of the adrenergic agent (s) from the matrix.

The materials from which the product is made may be selected to be stable during the release period for the drug delivery device. The materials may optionally be selected so that, after the drug delivery device has released the adrenergic agent(s) for a predetermined amount oftime, the drug delivery device erodes *in situ,* i.e., is bioerodible. The materials may also be selected so that, for the desired life of the delivery device, the materials are stable and do not significantly erode, and the pore size of the materials does not change. In certain embodiments using a matrix with the drug core, the matrix is bioerodible, while in other embodiments the matrix is non-bioerodible.

There are at least two functions of matrix material selected for the inner drug-containing core: to permit the ease manufacture of the core whether by compression, extrusion, co-extrusion or some other process; and to inhibit, or prevent, decomposition of the drug in the core due to the migration into the matrix of biological molecules. The matrix material of the inner drug-containing core inhibits, and preferably prevents, the passage of enzymes, proteins, and other materials into the drug-containing core that would lyse the drug before it has an opportunity to be released from the

device. As the core empties, the matrix may weaken and break down. Then, the outer layer will be exposed to degradation from both the outside and inside from water and enzymatic action. Drugs having higher solubilities are preferably linked to form low solubility conjugates; alternatively, drugs may be linked together to form molecules large enough or sufficiently insoluble to be retained in the matrix.

In addition to one or more adrenergic agents and matrix-forming polymers, the inner drug-containing core may include any biomaterials such as lipids (including long chain fatty acids) and waxes, anti-oxidants, and in some cases, release modifiers (e.g., water). These materials should be biocompatible and remain stable during the manufacturing process. In certain embodiments, the blend of active drugs, polymers, and biomaterials should be extrudable under desired processing conditions. The matrix-forming polymers or any biomaterials used should be able to carry a sufficient amount of active drug or drugs to produce therapeutically effective actions over the desired period of time. It is also preferred that the materials used as drug carriers have no deleterious effect on the activity of the adrenergic agent(s).

In certain embodiments, the matrix polymer(s) may be selected so that the release rate of the drug(s) from the matrix is determined, at least in part, by the physico-chemical properties of the drug(s), and not by the properties of the matrix. The pH of the matrix may also be selected such that it modifies the release rate of the drug(s). For example, where a drug is in free-base form, the matrix may include basic moieties, e.g., having a pKa that is higher than that of the drug, thereby slowing the protonation rate and ultimately the release rate of the drug. The matrix may also have moieties having a pKa that is less than but relatively close to that of the free base drug. In either of such embodiments, the matrix functions as a buffer to the protonation of the free base drug and, ultimately, to its release from the device. In addition, the pH microenvironment of the matrix may be varied by the addition of basic additives or by the use of phosphate or other standard buffers, thereby controlling the protonation of the drug(s) and its diffusion from the matrix. In certain embodiments, the matrix material is selected so that sustained release of the drug is controlled by the rate of protonation of the free-base drug, such that the drug's diffusion through the matrix has little or no effect on the drug's release rate from the matrix.

In certain embodiments, drug(s) may also be included in the outer layer. This may provide biphasic release with an initial burst such that when such a system is first placed in the body, a substantial fraction of the total drug released is released from the outer layer. Subsequently, more drug is released from the inner drug-containing core. The drug(s) included in the outer layer may be the same drug(s) as inside the core, including one or more adrenergic agents. Alternatively, the drugs included in the outer layer may be different from the drug(s) included in the core.

As noted in certain examples of the co-extruded embodiment described herein, it will be appreciated that a variety of materials may be used for the outer layer to achieve different release rate profiles. For example, as discussed in the aforementioned '972 patent, an outer layer may be surrounded by a permeable or impermeable additional layer, or may itself be formed of a permeable or semi-permeable material. Accordingly, co-extruded devices of the present invention may be provided with one or more outer layers using techniques and materials fully described in the '972 patent. Through the use of permeable or semi-permeable materials, drug(s) in the core may be released at various rates. In addition, even materials considered to be impermeable may permit release of drug(s) or other active agents in the core under certain circumstances. Thus, permeability of the outer layer may contribute to the release rate of a drug(s) over time, and may be used as a parameter to control the release rate over time for a deployed device.

In certain embodiments, the agent has a permeability coefficient in the outer layer of less than about $1x10^{-10}$cm/s. In other embodiments the permeability coefficient in the outer layer is greater than $1x10^{-10}$ cm/s, or even greater than $1x10^{-7}$ cm/s. In certain embodiments the permeability coefficient is at least $1x10^{-5}$ cm/s, or even at least $1x10^{-3}$ cm/s, or at least $1x10^{-2}$ cm/s.

Further, devices may be segmented into devices having, for example, an impermeable outer layer surrounding an inner drug-containing core, with each segment being optionally further coated by a semi-permeable or permeable layer to control a release rate through the exposed ends thereof. Similarly, the outer layer, or one or more additional layers surrounding the device, may be bioerodible at a known rate, so that core material is exposed after a certain period of time along some or all of the length of the tube, or at one or both ends thereof. Thus, it will be appreciated that, using various materials for the outer layer and one or more additional layers surrounding a co-extruded device, the delivery rate for the deployed device may be controlled to achieve a variety of release rate profiles.

As more fully described in U.S. Provisional Application No. 60/483,316, certain embodiments provide a polymer drug delivery system ("polymer system") comprising an inner core or reservoir ("inner core") that contains a therapeutically effective amount of an agent, a first coating layer that is impermeable, negligibly or partially permeable to the agent and, optionally, a second coating layer that is permeable or semi-permeable to the agent. Additional layers may also optionally be used.

In certain embodiments, the inner drug-containing core has biocompatible fluid and biocompatible solid components, where the biocompatible solid is less soluble in physiological fluid than in the biocompatible fluid. The biocompatible fluid may be hydrophilic, hydrophobic or amphiphilic; may be polymeric or nonpolymeric. Such fluid may also be a biocompatible oil. In certain embodiments, a biocompatible solid (e.g., a bioerodible polymer) is dissolved, suspended, or dispersed in the biocompatible fluid (to form a "biocompatible core component"). At least one agent, such as an

adrenergic agent, is also dispersed, suspended, or dissolved in the biocompatible core component.

The first coating layer surrounds the inner core, is an impermeable, negligibly or partially permeable polymer, and may feature one or more diffusion ports or pores ("ports") that further allow the drug to diffuse from the core out of the system. The rate of drug release from such systems may be controlled by the permeability of a drug matrix in the inner core (as described below), the solubility of the agent in the biocompatible core component, the thermodynamic activity of the agent in the biocompatible core component, the potential gradient of the agent from the inner core to the biological fluid, the size of the diffusion port(s), and/or the permeability of the first or second coating layer.

The first coating layer includes at least one polymer and is preferably bioerodible, but it may alternatively be non-bioerodible. The first coating layer covers at least part but preferably not all of the surface of the inner core, leaving at least one opening as a diffusion port through which the agent can diffuse. If a second coating layer is used, it may partially cover or cover essentially all of the first coating layer and inner core, and its permeability to the agent permits the agent to diffuse into the surrounding fluid. The first coating, in addition to or as an alternative to providing one or more diffusion ports, may further comprise a non-polymeric component that erodes *in vivo,* or it may comprise two or more different polymers (*e.g.*, having different monomer units, different molecular weights, different degrees of crosslinking, and/or different molar ratios of monomer units), at least one of which erodes *in vivo,* such that after implantation the first coating itself is capable of developing release ports that permit diffusion of the active agent.

A variety of materials may be suitable to form the coating layer(s) of these embodiments of the present invention. Preferable polymers are largely insoluble in physiological fluids. Suitable polymers may include naturally occurring or synthetic polymers. Certain exemplary polymers include, but are not limited to, PVA, cross-linked polyvinyl alcohol, cross-linked polyvinyl butyrate, ethylene ethylacrylate copolymer, polyehtyl hexylacrylate, polyvinyl chloride, polyvinyl acetals, plasticized ethylene vinylacetate copolymer, ethylene vinylchloride copolymer, polyvinyl esters, polyvinyl butyrate, polyvinylformal, polyamides, polymethylmethacrylate, polybutylmethacrylate, plasticized polyvinyl chloride, plasticized nylon, plasticized soft nylon, plasticized polyethylene terephthalate, natural rubber, polyisoprene, polyisobutylene, polybutadiene, polyethylene, polytetrafluoroethylene, polyvinylidene chloride, polyacrylonitrile, cross-linked polyvinylpyrrolidone, polytrifluorochloroethylene chlorinated polyethylene, poly(1,4-isopropylidene dipehenylene carbonate), vinylidene chloride, acrylonitrile copolymer, vinyl-chloride-diethyl fumerale copolymer, silicone rubbers, medical grade polydimethylsiloxanes, ethylene-propylene rubber, silicone-carbonate copolymers, vinylidene chloride-vinyl chloride copolymer, vinyl chloride-acrylonitrile copolymer, and vinylidene chloride-acrylonitride copolymer.

As noted above, where applied, the biocompatible core component includes at least one biocompatible solid (e.g., a bioerodible polymer) that is at least partially dissolved, suspended, or dispersed in a biocompatible polymeric or nonpolymeric fluid or a biocompatible oil. Further, the biocompatible solid is more soluble in the biocompatible fluid or oil than the physiological fluid such that, when the device is placed in contact with physiological fluid, the biocompatible core component precipitates or undergoes a phase transition. The inner core may be delivered as a gel. It may preferably be delivered as a particulate or a liquid that converts to a gel upon contact with water or physiological fluid. In some embodiments the nonpolymeric fluid may include a drug in free base form.

In certain embodiments, the biocompatible fluid of the biocompatible core component is hydrophilic (e.g., PEG, cremophor, polypropylene glycol, glycerol monooleate, and the like), hydrophobic, or amphiphilic. In certain embodiments, said fluid may be a monomer, polymer or a mixture of the same. If used, the biocompatible oil may be sesame oil, miglyol, or the like.

In certain embodiments, injectable liquids may be used that, upon injection, undergo a phase transition and are transformed *in situ* into gel delivery vehicles. In certain embodiments, at least one polymer in the inner core may convert from a drug-containing liquid phase to a drug-infused gel phase upon exposure to a physiological fluid. Technologies based on *in situ* gelling compositions are described in U.S. Patent Nos. 4,938,763, 5,077,049, 5,278,202, 5,324,519, and 5,780,044, all of which may be adapted to such embodiments of the present invention. In certain embodiments, the biocompatible solid of the biocompatible core component may be, for example, but without limitation, PLGA. In certain embodiments, the inner core is a viscous paste containing at least 10% agent, or preferably over 50% agent or, more preferably, over 75% agent.

In certain embodiments, the inner core comprises an *in situ* gelling drug delivery formulation comprising: (a) one more adrenergic agents; (b) a liquid, semi-solid, or wax PEG; and (c) a biocompatible and bioerodable polymer that is dissolved, dispersed, or suspended in the PEG. The formulation may optionally also contain additives, such as pore-forming agents (e.g., sugars, salts, and water-soluble polymers), and release rate modifiers (e.g., sterols, fatty acids, glycerol esters, and the like). As more fully described in U.S. Provisional Patent Application No. 60/482,677, such formulation, on contact with water or bodily fluids, undergoes exchange of the PEG for water, resulting in precipitation of both the polymer and the drug and subsequent formation of a gel phase within which the drug is incorporated. The drug subsequently diffuses from the gel over an extended period of time.

A "liquid" PEG is a polyethylene glycol that is a liquid at 20-30° C and ambient pressure. In certain preferred embodiments, the average molecular weight of the liquid PEG is between about 200 and about 400 g/mol. The PEG may be linear or it may be a bioabsorbable branched PEG, for example as disclosed in U.S. Patent Application No.

2002/0032298. In certain alternative embodiments, the PEG may be a semi-solid or wax, in which case the molecular weight will be larger, for example 3,000 to 6,000 amu. It will be understood that compositions comprising semi-solid and waxy PEGs may not be amenable to injection, and will accordingly be implanted by alternative means.

In certain embodiments, the adrenergic agent(s) is dissolved in PEG, while in other embodiments, the drug is dispersed or suspended in PEG in the form of solid particles. In yet other embodiments, the drug may be encapsulated or otherwise incorporated into particles, such as microspheres, nanospheres, liposomes, lipospheres, micelles, and the like, or it may be conjugated to a polymeric carrier. Any such particles are preferably less than about 500 microns in diameter, more preferably less than about 150 microns.

The polymer that is dissolved, dispersed, or suspended in PEG of the formulation discussed above may be any biocompatible PLGA polymer that is soluble in or miscible with PEG, and is less soluble in water. It is preferably water-insoluble, and is preferably a bioerodable polymer. The carboxyl termini of the lactide- and glycolide-containing polymer may optionally be capped, e.g., by esterification, and the hydroxyl termini may optionally be capped, e.g., by etherification or esterification. Preferably, the polymer is PLGA having a lactide:glycolide molar ratio of between 20:80 and 90:10, more preferably between 50:50 and 85:15.

The term "bioerodible" is synonymous with "biodegradable" and is art-recognized. It includes polymers, compositions and formulations, such as those described herein, that degrade during use. Biodegradable polymers typically differ from non-biodegradable polymers in that the former may be degraded during use. In certain embodiments, such use involves in vivo use, such as in vivo therapy, and in other certain embodiments, such use involves in vitro use. In general, degradation attributable to biodegradability involves the degradation of a biodegradable polymer into its component subunits, or digestion, e.g., by a biochemical process, of the polymer into smaller, non-polymeric subunits. In certain embodiments, biodegradation may occur by enzymatic mediation, degradation in the presence of water and/or other chemical species in the body, or both.

The terms "biocompatible " and "biocompatibility" when used herein are art-recognized and mean that the referent is neither itself toxic to a host (e.g., an animal or human), nor degrades (if it degrades) at a rate that produces byproducts (e.g., monomeric or oligomeric subunits or other byproducts) at toxic concentrations, causes inflammation or irritation, or induces an immune reaction, in the host. It is not necessary that any subject composition have a purity of 100% to be deemed biocompatible. Hence, a subject composition may comprise 99%, 98%, 97%, 96%, 95%, 90% 85%, 80%, 75% or even less of biocompatible agents, e.g., including polymers and other materials and excipients described herein, and still be biocompatible.

In certain embodiments, a polymer system is injected or otherwise inserted into a physiological system (e.g., a patient). Upon injection or other insertion, the polymer system will contact water or other immediately surrounding physiological fluid that will enter the polymer system and contact the inner core. In certain embodiments, the core materials may be selected so as to create a matrix that reduces (and thereby allows control of) the rate of release of the agent from the polymer system.

In preferred embodiments, the agent's rate of release from the polymer system is limited primarily by the permeability or solubility of the agent in the matrix. However, the release rate may be controlled by various other properties or factors. For example, but without limitation, the release rate may be controlled by the size of the diffusion port(s), the permeability of the second coating layer of the polymer system, the physical properties of the inner core, the dissolution rate of the inner core or components of said core, or the solubility of the agent in the physiological fluid immediately surrounding the polymer system.

In certain embodiments, the rate of release of the agent may be limited primarily by any of the foregoing properties. For example, in certain embodiments, the rate of release of the agent may be controlled, or even limited primarily by, the size of the diffusion port(s). Depending on the desired delivery rate of the agent, the first coating layer may coat only a small portion of the surface area of the inner core for faster release rates of the agent (i.e., the diffusion port(s) is relatively large), or may coat large portions of the surface area of the inner core for slower release rates of the agent (i.e., the diffusion port(s) is relatively small).

For faster release rates, the first coating layer may coat up to about 10% of the surface area of the inner core. In certain embodiments, approximately 5-10% of the surface area of the inner core is coated with the first coating layer for faster release rates.

Certain embodiments may achieve desirable sustained release if the first coating layer covers at least 25% of the surface area of the inner core, preferably at least 50% of the surface area, more preferably at least 75%, or even greater than 85% or 95% of the surface area. In certain embodiments, particularly where the agent is readily soluble in both the biocompatible core component and the biological fluid, optimal sustained release may be achieved if the first coating layer covers at least 98% or 99% of the inner core. Thus, any portion of the surface area of the inner core, up to but not including 100%, may be coated with the first coating layer to achieve the desired rate of release of the agent.

The first coating layer may be positioned anywhere on the inner core, including, but not limited to, the top, bottom, or any side of the inner core. In addition, it could be positioned on the top and a side, or the bottom and a side, or the top and the bottom, or on opposite sides or on any combination of the top, bottom, or sides. As described herein,

the coating layer may also cover the inner core on all sides while leaving a relatively small uncovered place as a port.

The composition of the first coating layer is selected so as to allow the above-described controlled release. The preferred composition of the first layer may vary depending on such factors as the active agent, the desired rate of release of the agent and the mode of administration. The identity of the active agent is important because its molecular size may determine, at least in part, its rate of release into the second coating layer if used.

In certain of such embodiments, the release rate of the agent from the inner core may be reduced by the permeability of the second coating layer. In certain embodiments, the second coating layer is freely permeable to the agent. In certain embodiments, the second coating layer is semi-permeable to the agent. In certain embodiments, the agent has a permeability coefficient in the second coating layer of less than about $1x10^{-10}$ cm/s. In other embodiments the permeability coefficient in the second coating layer is greater than $1x10^{-10}$ cm/s, or even greater than $1x10^{-7}$ cm/s. In certain embodiments the permeability coefficient is at least $1x10^{-5}$ cm/s, or even at least $1x10^{-3}$ cm/s, or at least $1x10^{-2}$ cm/s in the second layer.

In certain embodiments, the inner core undergoes a phase change and converts to a gel upon insertion of the polymer system in a physiological system. The phase change may reduce the rate of release of the agent from the inner core. For example, where at least part of the inner core is provided first as a liquid and converts to a gel, the gel phase of the biocompatible core component may be less permeable to the agent than is the liquid phase. In certain embodiments, the biocompatible core component in gel phase is at least 10% or even at least 25% less permeable to the agent than is the liquid phase. In other embodiments, the precipitated biocompatible solid is at least 50% or even at least 75% less permeable to the agent than is the biocompatible fluid. In certain embodiments, interaction of the inner core with the physiological fluid may alter the solubility of the agent in the core. For example, the inner core is at least 10% or even at least 25% less solubilizing to the agent than before interaction with physiological fluid. In other embodiments, the gel phase is at least 50% or even at least 75% less solubilizing.

In certain embodiments, the rate at which the biocompatible solid and/or fluid components of the inner core dissolve may impact the rate of release of the agent. In certain embodiments, as the biocompatible core component erodes or dissolves, the rate of release of the agent may increase. For example, less than about 10% of the biocompatible core component may erode over a period of about 6 hours. This may increase the rate of release of the agent by less than about 10% over that time. In certain embodiments, the biocompatible core component may erode or dissolve more slowly (e.g., less than about 10% over a period of about 24 hours, or even over a period of multiple days, weeks, or even months). In certain embodiments, such erosion may occur more rapidly (e.g., greater than about 10% over a period of about 6 hours, in certain embodiments even greater than 25% over a period of about 6 hours).

In certain embodiments, the release rate of the agent from the inner core may be controlled by the ratio of the agent to the biocompatible solid component of the core (also referred to as the "drug loading"). By changing the drug loading, different release rate profiles can be obtained. Increasing the drug loading may increase the release rate. For a slower release profile, drug loading may be less than 10%, and preferably less than 5%. For a faster release profile, drug loading may be more than 10%, and preferably more than 20%, or even greater than 50%.

Thus, the rate of release of the agent according to the invention may be limited primarily by any of the above properties or any other factor. For example, but without limitation, the release rate may be controlled by the size and/or location of the diffusion port(s), the permeability or other properties of the first or a second coating layer in the polymer system, the physical properties of the inner core, the dissolution rate of the biocompatible core component, the solubility of the agent within the inner core, the solubility of the agent in the physiological fluid immediately surrounding the polymer system, etc.

The phrase "limited primarily by" when used herein refers to the factor(s) associated with the rate-determining step in the release rate of an agent from the inventive system. For example, but without limitation, where the rate of release (e.g., the rate-determining step) is a result of a property of the matrix (e.g., size of the diffusion port), the rate of release is also said to be "limited primarily by" such property. In some embodiments, the devices of the present invention utilize a sustained-release formulation containing a therapeutically effective amount of at least one adrenergic agent. Such formulations are more fully described in U.S. Provisional Patent Application No. 60/442,499. In such embodiments, it is preferred that the adrenergic agent be a free base that is provided, for example, as a hydrophobic viscous oil. As used herein, the term "free base" means an agent with a basic nitrogen moiety that exists primarily in protonated (salt) form if the agent is dissolved in water. The free base has a conjugate acid with a pKa greater than about 4 and less than about 14, preferably greater than about 5 and less than about 12. Without limitation, moieties that typically include a basic nitrogen are amines, hydrazines, anilines, pyridines, amidines, and guandines.

In other formulations of such embodiments, the therapeutic agent is a protonated acid. As used herein, the term "protonated acid" means an agent having a moiety capable of being deprotonated in aqueous solution to form a salt, where the moiety has a pKa greater than about 4 but less than about 14, preferably greater than about 5 but less than about 12. Without limitation, exemplary acidic moieties include carboxylate, phosphate, sulfonamide, thiol, imidazole, and imide.

The adrenergic agent in its salt form (e.g., the unprotonated form of the protonated acid and the protonated

form of the free-base) is preferably highly soluble in water, whereas the agent itself, e.g., protonated acid or free-base, preferably has a low solubility in water.

As discussed herein, an agent in free-base form is referred to as being in "uncharged" or "charge-neutral" form; when protonated, such an agent is referred to as being in "charged," "protonated," or "salt" form. Analogously, a protonated acid agent is referred to as being in "uncharged" or "charge-neutral" form; in its deprotonated form, such an agent is referred to as being in "charged," "deprotonated," or "salt" form.

Without wishing to be bound by any particular mechanism, it is expected that release of a free-base agent occurs at a given physiologic site as the free base diffuses from the inner core of a sustained-release device of the present invention and becomes protonated in the physiological fluid. Upon protonation, the agent dissolves in the surrounding fluid. In embodiments utilizing a protonated acid, it is expected that the release of the agent occurs as the acid diffuses from the inner core and becomes deprotonated in the physiological fluid, whereupon the agent dissolves rapidly into the fluid. In either embodiment, it is expected that the rate of release of the agent is controlled more by the rate of ionization of the agent (e.g., rate of protonation of the free base or rate of deprotonation of the protonated acid) than by the rate of the agent's diffusion from the inner core or the rate of the charged agent's dissolution in the immediately surrounding fluid.

In certain embodiments, the coating layer(s) may be formed with the adrenergic agent(s) as a substantially homogeneous system, formed by mixing one or more suitable monomers with the agent(s), then polymerizing the monomer to form a polymer system. In this way, the agent is dissolved or dispersed in the polymer. In other embodiments, the agent is mixed into a liquid polymer or polymer dispersion and then the polymer is further processed to form the inventive coating(s). Suitable further processing may include crosslinking with suitable crosslinking agents, further polymerization of the liquid polymer or polymer dispersion, copolymerization with a suitable monomer, block copolymerization with suitable polymer blocks, etc. The further processing traps the agent in the polymer so that the agent is suspended or dispersed in the polymer system.

In certain embodiments, the solubility in water of the uncharged form of the agent is less than 10 mg/ml, or even less than 1.0 mg/ml, 0.1 mg/ml, 0.01 mg/ml or 0.001 mg/ml. In certain embodiments, the agent in its salt form is at least 10 times more soluble in water relative to the uncharged form, or even at least 100, 1000 or preferably 10,000 times more soluble in water relative to the uncharged form of the agent.

Suitable adrenergic agents include but are not limited to brimonidine, apraclonidine, bunazosin, timolol, betaxolol, levobetaxolol, levobunalol, carteolol, isoprenaline, fenoterol, metipranolol, clenbuterol, epinephrine, and dipivefrin.

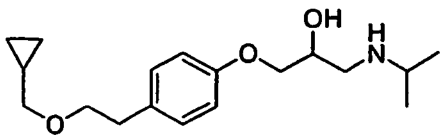

Betaxolol

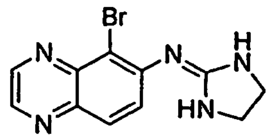

Brimonidine

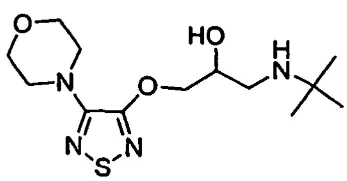

Timolol

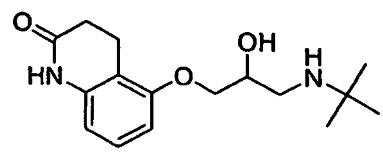

Carteolol

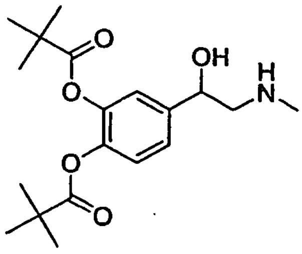

Dipivefrin

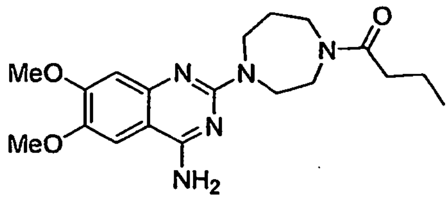

Bunazosin

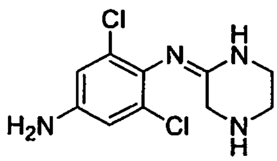

Apraclonidine

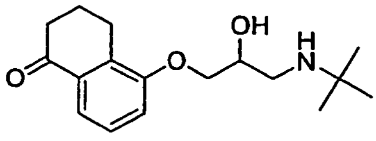

Levobunolol

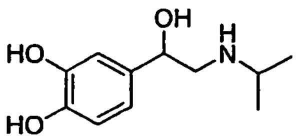

Isoprenaline

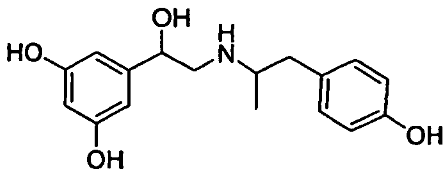

Fenoterol

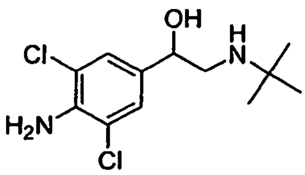

Clenbuterol

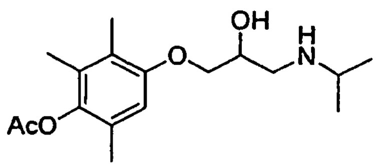

Metipranolol.

Further examples of suitable adrenergic agents are propranolol, isoproterenol, atenolol, carvediol, metoprolol, nadolol, sotalol, befunolol, penbutolol, labetalol, and nipradolol. Cardiac and pulmonary beta blockers, Propranolol, labetalol, and isoproterenol may also be used herewith.

Another embodiment of the present invention provides a sustained-release drug delivery device adapted for insertion into or adjacent to the eye of a patient, where the drug delivery device comprises:

(i) an inner drug core comprising at least one adrenergic agent and a polymer matrix material, wherein the carbonic anhydrase inhibitor(s) is/are admixed in the polymer matrix material to inhibit or prevent decompostion of the carbonic anhydrase inhibitor(s);
(ii) a first coating that is impermeable to the passage of the at least one adrenergic agent, having one or more openings therein through which the at least one adrenergic agent can diffuse, and which is substantially insoluble and inert in body fluids and compatible with body tissues; and
(iii) one or more additional coatings that are permeable to the passage of the at least one adrenergic agent, and which are substantially insoluble and inert in body fluids and compatible with body tissues;

wherein the impermeable and permeable coatings are disposed about the inner core so as to produce, when inserted,

a constant rate of release of the at least one adrenergic agent from the device. Such a sustained-release device is disclosed in U.S. Pat. No. 5,378,475.

While embodiments of the device described in the '475 Patent solve many of the problems pertaining to drug delivery, polymers suitable for coating the inner core are frequently relatively soft and technical difficulties can arise in the production of uniform films. This is especially true when attempting to coat non-spherical bodies with edges, such as those having a cylindrical shape. In such cases, relatively thick films must be applied to achieve uninterrupted and uniform coatings, which adds significant bulk to the device. Alternatively, the added bulk of the film coating can be accommodated by limiting the internal volume of the device, but this limits the amount of drug that can be delivered, potentially limiting both efficacy and duration.

The issue of device size is extremely important in the design of devices for insertion into or in the vicinity of the eye. Larger devices require more complex procedures to both insert and remove, and involve an associated increased risk of complications, longer healing or recovery periods, and potential side effects.

The aforementioned U.S. Patent No. 5,902,598 presents solutions to the problems of manufacturing devices that are small enough for insertion into or in the vicinity of the eye, by loading a drug composition into a preformed shell rather than attempting to coat the drug core, but manufacturing difficulties can arise with this method. In particular, the impermeable inner coating layer that immediately surrounds the drug reservoir is typically so thin that the shell is not capable of supporting its own weight. While beneficial from the standpoint of reducing the size of the device while still sealing the drug reservoir, the relative flaccidity of this inner layer makes it difficult to load the reservoir with a drug. Because this inner layer does not have the dimensional stability or structural strength to accept the introduction of a drug core without changing shape, a relatively solid drug or drug-containing mixture must be used in order to manufacture the device. Loading a drug slurry into an inner layer that does not hold its own shape results in the combination of the drug slurry and inner layer being extremely difficult to handle during manufacture without damaging it, because the inner layer collapses and the drug-containing mixture flows out. An illustrative analogy may be made to the task of filling a plastic bag with water.

As more fully described in U.S. Patent No. 6,375,972, yet another embodiment of the present invention addresses these problems by providing a sustained-release drug delivery system comprising an inner reservoir containing a drug core comprising at least one adrenergic agent, and an inner tubular covering that is substantially impermeable to the passage of the drug and that covers at least a portion of the drug core. The term "substantially impermeable," as used herein, means that the layer will not allow passage of the adrenergic agent(s) at a rate sufficient to affect intraocular pressure if it completely covers the drug core. Conversely, a permeable layer will allow passage of the adrenergic agent (s) from the device at a rate that is sufficient to affect intraocular pressure. It will be appreciated that the invention operates on the premise that diffusion through the permeable layer(s) is faster than diffusion through the substantially impermeable layer.

The inner tubular covering is sized and formed of a material so that it is capable of supporting its own weight, and has first and second ends such that the tubular covering and the two ends define, an interior space for containing a drug reservoir. A substantially impermeable member is positioned at the first end, said impermeable member preventing passage of the adrenergic agent(s) out of the reservoir through the first end, and a permeable member is positioned at the second end, which allows diffusion of the adrenergic agent(s) out of the reservoir through the second end.

The drug reservoir of such embodiments occupies a space defined by the tubular wall of the device and its termini. The reservoir may be filled with one or more fluid drug core compositions; including, but not limited to, solutions, suspensions, slurries, pastes, or other non-solid drug formulations containing a adrenergic agent(s). The reservoir may also be filled with a non-fluid (e.g., a gum, gel, or solid) drug core comprising at least one adrenergic agent.

In any event, it will be appreciated that as the adrenergic agent(s) is released from the device over time, a non-fluid drug core that physically erodes as the drug dissolves away will not continue to fully occupy the reservoir volume. Applicants have found that a tube that has dimensional stability and is capable of supporting its own weight can accept a drug core therein without changing shape, and retain its structural integrity as the drug is released. Because the reservoir is defined by a relatively rigid tubular shell, the reservoir will maintain its shape and size, and so the regions of the device through which drug diffusion takes place will not change in area. As described in the equations below, constant diffusion area favors a constant rate of drug release.

The use of a sufficiently rigid tube of material to hold the drug reservoir during manufacture also makes for significantly easier handling of the tube and reservoir, because the tube fully supports both its own weight and the weight of the reservoir even when the reservoir is not solid. The pre-formed tube used in the present invention is not a simple coating, because a coating is typically not pre-formed and cannot support its own weight. Also, the rigid structure of such embodiments allows the use of drug slurries drawn into the tube, which facilitates the fabrication of longer cylindrical devices. Furthermore, because of the relative ease of manufacturing devices in accordance with such embodiments, more than one reservoir, optionally containing more than one drug, can be incorporated into a single device.

During use the invention, although the size and/or shape of the drug core may change as drug dissolves and diffuses out of the device, the tube that defines the volume of the drug reservoir is sufficiently strong or rigid to maintain

a substantially constant diffusion area, so that the diffusion rate from the device does not change substantially despite dimensional changes in the drug core. By way of example and not of limitation, an exemplary method of ascertaining if the tube is sufficiently rigid is to form a device in accordance with the present invention, and to measure the diffusion rate of the drug from the device over time. If the diffusion rate changes more than 50% from the diffusion rate expected based on the chemical potential gradient across the device at any particular time, the tube has changed shape and is not sufficiently rigid. Another exemplary test is to visually inspect the device as the drug diffuses over time, looking for signs that the tube has collapsed in part or in full.

The use of permeable and impermeable tubes in accordance with the present invention provides resistance to reverse flow, i.e., flow back into the device. The tube or tubes assist in preventing large proteins from binding, solubilizing, or degrading the adrenergic agent(s) before it leaves the drug reservoir. Also, the tube or tubes assist in preventing oxidation and protein lysis, as well as preventing other biological agents from entering the reservoir and degrading the contents.

It will be understood that "reservoir" generally refers to the inner volume of the device in the sense that it acts as a container, and "core" generally refers to the contents of the container. However, the terms "core" and "reservoir" are occasionally used interchangeably in describing the devices of the invention, because as initially manufactured the drug core and the drug reservoir that contains it are essentially co-extensive. As the device delivers the adrenergic agent (s) during use, however, a solid drug core may gradually erode, and no longer be co-extensive with the drug reservoir that contains it.

In preferred embodiments, the subject invention provides methods and compositions for treating or reducing the risk of disease or other physiological conditions, such as glaucoma. The invention particularly contemplates sustained-release compositions for systemic delivery of therapeutic agents that are highly water-soluble in their salt forms. In preferred embodiments, such highly water-soluble agents include anti-glaucoma agents such as betaxolol hydrochloride or timolol maleate.

Turning now to the drawing figures, FIG. 1 illustrates a longitudinal cross-sectional view of a drug delivery device 100 in accordance with the present invention. Device 100 includes an outer layer 110, an inner tube 112, a reservoir or drug core 114, and an inner cap 116. Outer layer 110 is preferably a permeable layer, that is, the outer layer is permeable to the adrenergic agent(s) contained within reservoir 114. Cap 116 is positioned at one end of tube 112. Cap 116 is preferably formed of a substantially impermeable material, that is, the cap is not permeable to the adrenergic agent(s) contained within reservoir 114. Cap 116 is joined at end 118, 120 of inner tube 112, so that the cap and the inner tube together close off a space in the tube in which reservoir 114 is positioned. Inner tube 112 and cap 116 can be formed separately and assembled together, or the inner tube and the cap can be formed as a single, integral, monolithic element.

Outer layer 110 at least partially, and preferably completely, surrounds both tube 112 and cap 116, as illustrated in FIG. 1. While it is sufficient for outer layer 110 to only partially cover tube 112 and cap 116, and in particular the opposite ends of device 100, the outer layer is preferably formed to completely envelop both the tube and cap to provide structural integrity to the device, and to facilitate further manufacturing and handling because the device is less prone to break and fall apart. While FIG. 1 illustrates cap 116 having an outer diameter the same as the outer diameter of inner tube 112, the cap can be sized somewhat smaller or larger than the outer diameter of the inner tube while remaining within the spirit and scope of such embodiments of the present invention.

Reservoir 114 is positioned inside inner tube 112, as described above. A first end 122 abuts against cap 116, and is effectively sealed by the cap against the diffusion of drug through the first end. On the end of reservoir 114 opposite cap 116, the reservoir is preferably in direct contact with outer layer 110. As will be readily appreciated by one of ordinary skill in the art, as carbonic anhydrase inhibitor(s) is released from a non-fluid core contained within reservoir 114, the core may shrink or otherwise change shape, and therefore may not fully or directly contact outer layer 110 at the end of the reservoir opposite cap 116. As outer layer 110 is permeable to the adrenergic agent(s) in reservoir 114, the drug is free to diffuse out of the reservoir along a first flow path 124 into portions of outer layer 110 immediately adjacent to the open end of the reservoir. From outer layer 110, the drug is free to diffuse along flow paths 126 out of the outer layer and into the tissue or other anatomical structure in which device 100 is inserted. Optionally, holes can be formed through inner layer 112 to add additional flow paths 126 between reservoir 114 and permeable outer layer 110.

FIG. 1 illustrates only the positions of the several components of device 100 relative to one another, and for ease of illustration shows outer layer 110 and inner tube 112 as having approximately the same wall thickness. The thickness of the layer and wall are exaggerated for ease of illustration, and are not drawn to scale. While the walls of outer layer 110 and inner tube 112 may be of approximately the same thickness, the inner tube's wall thickness can be significantly thinner or thicker than that of the outer layer within the spirit and scope of the present invention. Additionally, device 100 is preferably cylindrical in shape, for which a transverse cross-section (not illustrated) will show a circular cross-section of the device. While it is preferred to manufacture device 100 as a cylinder with circular cross-sections, it is also within the scope of the invention to provide cap 116, adrenergic agent(s) reservoir 114, inner tube 112, and/or outer layer 110 with other cross-sections, such as ovals, ellipses, rectangles, including squares, triangles, as well as

any other regular polygon or irregular shapes. Furthermore, device 100 can optionally further include a second cap (not illustrated) on the end opposite cap 116; such a second cap could be used to facilitate handling of the device during fabrication, and would include at least one through hole for allowing adrenergic agent(s) from reservoir 114 to flow from the device. Alternatively, the second cap may be formed of a permeable material.

Where the device is adapted for insertion into the lacrimal canaliculus, inner tube 112, 212, or 312 will be sized to fit within the lacrimal canaliculus, and will preferably be formed with a collarette, sized to rest on the exterior of the lacrimal punctum, at the end opposite cap 116, 242, or 316. It will be appreciated that permeable outer layer 110, 210, or 310 need not cover the entire device in this embodiment, as drug release will preferably be limited to the region of the device intended to remain external to the canaliculus.

FIG. 2 illustrates a device 200 in accordance with a second example of such embodiments of the present invention. Device 200 includes an impermeable inner tube 212, a adrenergic agent(s) drug core 214, and a permeable plug 216. Device 200 optionally and preferably includes an impermeable outer layer 210, which adds mechanical integrity and dimensional stability to the device, and aids in manufacturing and handling the device. As illustrated in FIG. 2, drug core 214 is positioned in the interior of inner tube 212, in a fashion similar to core 114 and inner tube 112 described above. Plug 216 is positioned at one end of inner tube 212, and is joined to the inner tube at end 218, 220 of the inner tube. While plug 216 may extend radially beyond inner tube 212, as illustrated in FIG. 2, the plug may alternatively have substantially the same radial extent as, or a slightly smaller radial extent than, the inner tube, while remaining within the scope of the invention. As plug 216 is permeable to the adrenergic agent(s) contained in the reservoir, the adrenergic agent(s) is free to diffuse through the plug from the reservoir. Plug 216 therefore must have a radial extent that is at least as large as the radial extent of reservoir 214, so that the primary diffusion pathway 230 out of the reservoir is through the plug. On the end of inner tube 212 opposite plug 216, the inner tube is closed off or sealed only by outer layer 210, as described below. Optionally, a substantially impermeable cap 242, which can take the form of a disc, is positioned at the end of reservoir opposite plug 216. When provided, cap 242 and inner tube 212 can be formed separately and assembled together, or the inner tube and the cap can be formed as a single, integral, monolithic element.

Outer tube or layer 210, when provided, at least partially, and preferably completely, surrounds or envelopes inner tube 212, adrenergic agent(s) reservoir 214, plug 216, and optional cap 242, except for an area immediately adjacent to the plug which defines a port 224. Port 224 is, in preferred embodiments, a hole or blind bore which leads to plug 216 from the exterior of the device. As outer layer 210 is formed of a material that is impermeable to the adrenergic agent(s) in reservoir 214, the ends of inner tube 212 and reservoir 214 opposite plug 216 are effectively sealed off, and do not include a diffusion pathway for the adrenergic agent(s) to flow from the reservoir. According to a preferred embodiment, port 224 is formed immediately adjacent to plug 216, on an end 238 of the plug opposite end 222 of reservoir 214. Plug 216 and port 224 therefore include diffusion pathways 230, 232, through the plug and out of device 200, respectively.

While port 224 in the embodiment illustrated in FIG. 2 has a radial extent that is approximately the same as inner tube 212, the port can be sized to be larger or smaller, as will be readily apparent to one of ordinary skill in the art. For example, instead of forming port 224 radially between portions 228, 230 of outer layer 210, these portions 228, 230 can be removed up to line 226, to increase the area of port 224. Port 224 can be further enlarged, as by forming outer layer 210 to extend to cover, and therefore seal, only a portion or none of the radial exterior surface 240 of plug 216, thereby increasing the total surface area of port 224 to include a portion or all of the outer surface area of the plug.

In accordance with yet another embodiment of the invention, port 224 of device 200 can be formed immediately adjacent to radial external surface 240 of plug 216, in addition to or instead of being formed immediately adjacent to end 238 of the plug. As illustrated in FIG. 4, port 224 can include portions 234, 236, which extend radially away from plug 216. These portions can include large, continuous, circumferential and/or longitudinal portions 236 of plug 216 which are not enveloped by outer layer 210, illustrated in the bottom half of FIG. 4, and/or can include numerous smaller, circumferentially spaced apart portions 234, which are illustrated in the top half of FIG. 4. Advantageously, providing port 224 immediately adjacent to radial external surface 240 of plug 216, as numerous, smaller openings 234 to the plug, allows numerous alternative pathways for the adrenergic agent(s) to diffuse out of device 200 in the event of a blockage of portions of the port. Larger openings 236, however, benefit from a relative ease in manufacturing, because only a single area of plug 216 need be exposed to form port 224.

According to yet another embodiment of the invention, plug 216 is formed of a substantially impermeable material and outer layer 210 is formed of a permeable material. A hole or holes are formed, e.g., by drilling, through one or more of inner layer 212, cap 242, and plug 216, which permit adrenergic agent(s) to be released from reservoir 214 through outer layer 210. According to another embodiment, plug 216 is eliminated as a separate member, and permeable outer layer 210 completely envelopes inner tube 212 and cap 242 (if provided). Thus, the diffusion pathways 230, 232 are through outer layer 210, and no separate port, such as port 224, is necessary. By completely enveloping the other structures with outer layer or tube 210, the system 200 is provided with further dimensional stability. Further optionally, plug 216 can be retained, and outer layer 210 can envelop the plug as well.

According to yet another such embodiment of the present invention, inner tube 212 is formed of a permeable

material, outer layer 210 is formed of an impermeable material, and cap 242 is formed of either a permeable or an impermeable material. Optionally, cap 242 can be eliminated. As described above, as outer layer 210 is impermeable to the adrenergic agent(s) in reservoir 214, plug 216, port 224, and optional ports 234, 236, are the only pathways for passage of the adrenergic agent(s) out of device 200.

The shape of device 200 can be, in a manner similar to that described above with respect to device 100, any of a large number of shapes and geometries. Furthermore, both device 100 and device 200 can include more than one reservoir 114, 214, included in more than one inner tube 112, 212, respectively, which multiple reservoirs can include different adrenergic agents, or ocular medicaments such as a miotic agent in addition to a adrenergic agent, for diffusion out of the device. In device 200, multiple reservoirs 214 can be positioned to abut against only a single plug 216, or each reservoir 214 can have a dedicated plug for that reservoir. Such multiple reservoirs can be enveloped in a single outer layer 110, 210, as will be readily appreciated by one of ordinary skill in the art.

Turning now to FIG. 3, FIG. 3 illustrates a device 300 in accordance with a third exemplary embodiment of the invention. Device 300 includes a permeable outer layer 310, a substantially impermeable inner tube 312, a reservoir 314, a substantially impermeable cap 316, and a permeable plug 318. A port 320 communicates plug 318 with the exterior of the device, as described above with respect to port 224 and plug 216. Inner tube 312 and cap 316 can be formed separately and assembled together, or the inner tube and the cap can be formed as a single, integral, monolithic element. The provision of permeable outer layer 310 allows the adrenergic agent(s) in reservoir or drug core 314 to flow through the outer layer in addition to port 320, and thus assists in raising the overall delivery rate. Of course, as will be readily appreciated by one of ordinary skill in the art, the permeability of plug 318 is the primary regulator of the drug delivery rate, and is accordingly selected. Additionally, the material out of which outer layer 310 is formed can be specifically chosen for its ability to adhere to the underlying structures, cap 316, tube 312, and plug 318, and to hold the entire structure together. Optionally, a hole or holes 322 can be provided through inner tube 312 to increase the flow rate of adrenergic agent(s) from reservoir 314.

In order to maximize the useful life of the device, preferred formulations will be those that contain as large a mass of active agent as possible while retaining an effective rate of dissolution. By way of example, a dense, compressed solid that contains at least 90% of a non-salt form of a adrenergic agent would be a preferred drug core formulation.

A large number of materials can be used to construct the devices of the present invention. The only requirements are that they are inert, non-immunogenic, and of the desired permeability, as described herein.

In another embodiment, only a single outer layer need be used. FIG. 6 illustrates such an embodiment, wherein the sustained release device (product 612) includes an outer layer or skin 614 and an inner core 616.

Materials that may be suitable for fabricating devices 100, 200, , 300, and 712 include naturally occurring or synthetic materials that are biologically compatible with body fluids and/or eye tissues, and essentially insoluble in body fluids with which the material will come in contact. The use of rapidly dissolving materials or materials highly soluble in eye fluids are to be avoided since dissolution of the outer layers 110, 210, 310 would affect the constancy of the drug release, as well as the capability of the system to remain in place for a prolonged period of time.

Naturally occurring or synthetic materials that are biologically compatible with body fluids and eye tissues and essentially insoluble in body fluids with which the material will come in contact include, but are not limited to: ethyl vinyl acetate, polyvinyl acetate, cross-linked polyvinyl alcohol, cross-linked polyvinyl butyrate, ethylene ethylacrylate copolymer, polyethyl hexylacrylate, polyvinyl chloride, polyvinyl acetals, plasticized ethylene vinylacetate copolymer, polyvinyl alcohol, ethylene vinylchloride copolymer, polyvinyl esters, polyvinylbutyrate, polyvinylformal, polyamides, polymethylmethacrylate, polybutylmethacrylate, plasticized polyvinyl chloride, plasticized nylon, plasticized soft nylon, plasticized polyethylene terephthalate, natural rubber, polyisoprene, polyisobutylene, polybutadiene, polyethylene, polytetrafluoroethylene, polyvinylidene chloride, polyacrylonitrile, cross-linked polyvinylpyrrolidone, polytrifluorochloroethylene, chlorinated polyethylene, poly(1,4'-isopropylidene diphenylene carbonate), vinyl chloride-diethyl fumarate copolymer, silicone rubbers, especially the medical grade polydimethylsiloxanes, ethylene-propylene rubber, silicone-carbonate copolymers, vinylidene chloride-vinyl chloride copolymer, vinyl chloride-acrylonitrile copolymer, vinylidene chloride-acrylonitrile copolymer, gold, platinum, and (surgical) stainless steel.

Specifically, outer layer 210 of device 200 may be made of any of the above-listed polymers or any other polymer that is biologically compatible with body fluids and eye tissues, essentially insoluble in body fluids with which the material will come in contact, and permeable to the passage of the adrenergic agent(s).

When inner tube 112, 212, 312 is selected to be substantially impermeable, as described above, to the passage of the adrenergic agent(s) from the inner core or reservoir out to adjacent portions of the device, the purpose is to block the passage of the adrenergic agent(s) through those portions of the device, and thus limit the release of the adrenergic agent(s) from the device to selected regions of the outer layer and plugs 216 and 318.

The composition of outer layer 110, e.g., the polymer, is preferably selected so as to allow the above-described controlled release. The preferred composition of outer layer 110 and plug 216 will vary depending on such factors as the identity of the adrenergic agent(s), the desired rate of release, and the mode of implantation or insertion. The identity of the active agent is important since it determines the desired therapeutic concentration, and because the physico-chemical properties of the molecule are among the factors that affect the rate of release of the agent into and through the outer layer 110 and plug 216.

Caps 116, 242, 316 are substantially impermeable to the passage of the adrenergic agent(s) and may cover a portion of the inner tube not covered by the outer layer. The physical properties of the material, preferably a polymer, used for the caps can be selected based on their ability to withstand subsequent processing steps (such as heat curing) without suffering deformation of the device. The material, e.g., polymer, for substantially impermeable outer layer 210 can be selected based on the ease of coating inner tube 212. Cap 116 and inner tubes 112, 212, 312 can independently be formed of any of a number of materials, including PTFE, polycarbonate, polymethyl methacrylate, polyethylene alcohol, high grades of ethylene vinyl acetate (9% vinyl, content), and polyvinyl alcohol (PVA). Plugs 216, 318 can be formed of any of a number of materials, including cross-linked PVA, as described below.

Outer layers 110, 210, 310, and plugs 216, 318 of the device must be biologically compatible with body fluids and tissues, essentially insoluble in body fluids with which the material will come in contact, and outer layer 110 and plugs 216, 318 must be permeable to the passage of the adrenergic agent(s).

The adrenergic agent(s) diffuses in the direction of lower chemical potential, i.e., toward the exterior surface of the device. At the exterior surface of the device, equilibrium is again established. When the conditions on both sides of outer layer 110 or plugs 216, 318 are maintained constant, a steady state flux of the adrenergic agent(s) will be established in accordance with Fick's Law of Diffusion. The rate of passage of the drug through the material by diffusion is generally dependent on the solubility of the drug therein, as well as on the thickness of the wall. This means that selection of appropriate materials for fabricating outer layer 110 and plug 216 will be dependent on the particular adrenergic agent(s) to be used.

The rate of diffusion of the adrenergic agent(s) through a polymeric layer of the invention may be determined via diffusion cell studies carried out under sink conditions. In diffusion cell studies carried out under sink conditions, the concentration of drug in the receptor compartment is essentially zero when compared to the high concentration in the donor compartment. Under these conditions, the rate of drug release is given by:

$$Q/t=(D \cdot K \cdot A \cdot DC)/h$$

where Q is the amount of drug released, t is time, D is the diffusion coefficient, K is the partition coefficient, A is the surface area, DC is the difference in concentration of the drug across the membrane, and h is the thickness of the membrane.

In the case where the agent diffuses through the layer via water filled pores, there is no partitioning phenomenon. Thus, K can be eliminated from the equation. Under sink conditions, if release from the donor side is very slow, the value DC is essentially constant and equal to the concentration of the donor compartment. Release rate therefore becomes dependent on the surface area (A), thickness (h), and diffusivity (D) of the membrane. The surface area is a function of the size of the particular device, which in turn is dependent on the desired size of the adrenergic agent(s) drug core or reservoir.

Thus, permeability values may be obtained from the slopes of a Q versus time plot. The permeability P, can be related to the diffusion coefficient D, by:

$$P=(K \cdot D)/h$$

Once the permeability is established for the material permeable to the passage of the agent, the surface area of the agent that must be coated with the material impermeable to the passage of the agent may be determined. This may be done by progressively reducing the available surface area until the desired release rate is obtained.

Exemplary microporous materials suitable for use as outer layer 110 and plugs 216, 318, for instance, are described in U.S. Patent No. 4,014,335. These materials include but are not limited to cross-linked polyvinyl alcohol, polyolefins or polyvinyl chlorides or cross-linked gelatins; regenerated, insoluble, non-erodable cellulose, acylated cellulose, esterified celluloses, cellulose acetate propionate, cellulose acetate butyrate, cellulose acetate phthalate, cellulose acetate diethylaminoacetate; polyurethanes, polycarbonates, and microporous polymers formed by co-precipitation of a polycation and a polyanion modified insoluble collagen. Cross-linked polyvinyl alcohol is preferred for both outer layer 110 and plugs 216, 318. Preferred impermeable portions of the devices, e.g., cap 116 and inner tubes 112, 212, are formed ofPTFE or ethyl vinyl alcohol.

The drug delivery system of the present invention may be inserted into or adjacent to the eye via any of the

methods known in the art for ocular implants and devices. One or more of the devices may be administered at one time, or more than one agent may be included in the inner core or reservoir, or more than one reservoir may be provided in a single device.

Devices intended for insertion into the eye, for example into the vitreous chamber, may remain in the vitreous permanently after treatment is complete. Such devices may provide sustained release of the adrenergic agent(s) for a period of from several days to over five years. In certain embodiments, sustained release of the at least one agent may occur for a period of one or more months, or even greater than one or more years.

When such devices are prepared for insertion within the vitreous of the eye, it is preferred that the device does not exceed about 7 millimeters in any direction. Thus, the cylindrical devices illustrated in FIGS. 1 and 2 would preferably not exceed 7 millimeters in height or 3 millimeters in diameter, more preferably less than 1 mm in diameter and more preferably less than 0.5 mm in diameter. The preferred thickness of the walls of inner tubes 112, 212 ranges between about 0.01 mm and about 1.0 mm. The preferred thickness of the wall of outer layer 110 ranges between about 0.01 mm and about 1.0 mm. The preferred thickness of the wall of outer layer 210 ranges between about 0.01 mm and 1.0 mm. The inner drug-containing core of the various embodiments of the present invention preferably contains a high proportion of adrenergic agent(s), so as to maximize the amount of drug contained in the device and maximize the duration of drug release. Accordingly, in some embodiments, the drug core may consist entirely of one or more adrenergic agents in crystalline or amorphous form.

As noted above, the adrenergic agent(s) may be present in neutral form, or it may be in the form of a pharmaceutically acceptable salt, a codrug, or a prodrug. Where the adrenergic agent(s) comprises less than 100% of the core, suitable additives that may be present include, but are not limited to, polymeric matrices (e.g., to control dissolution rate or to maintain the shape of the core during use), binders (e.g., to maintain the integrity of the core during manufacture of the device), and additional pharmacological agents (e.g., a miotic agent or a PGF-2$\alpha$ analogue).

In some embodiments, the inner core is solid and is compressed to the highest density feasible, again to maximize the amount of contained drug. In alternative embodiments, the drug core may not be solid. Non-solid forms include, but are not limited to, gums, pastes, slurries, gels, solutions, and suspensions. It will be appreciated that the drug core may be introduced to the reservoir in one physical state and thereafter assume another state (e.g., a solid drug core may be introduced in the molten state, and a fluid or gelatinous drug core may be introduced in a frozen state).

The preferred rate of release of a given adrenergic agent will of course depend not only on the potency of the particular agent, but on the location of the device and the rate of clearance of the agent from the eye. Devices located within the eye will be less affected by loss of the adrenergic agent to lacrimal drainage and will not be limited by the rate of penetration of the agent through the cornea. As a result, such devices can maintain an effective concentration of drug in the ciliary processes with a lower release rate than can devices implanted external to the eye. Also, longer-acting adrenergic agents will require a lower release rate to maintain a therapeutically effective concentration.

Also described is a method for administering an adrenergic agent to a patient, comprising implanting the sustained release drug device described above into or adjacent to the eye of the patient.

## Claims

**1.** A sustained release drug delivery device adapted for insertion or implantation in or adjacent to the eye of a patient, the drug delivery device comprising:

(i) an inner drug core comprising at least one adrenergic agent and a polymer matrix, material, wherein the adrenergic agent(s) is/are admixed in the polymer matrix material to inhibit or prevent decomposition of the adrenergic agent(s);
(ii) a first layer on the surface of the inner drug core that is substantially impermeable to the passage of the at least one adrenergic agent, having one or more openings therein which permit diffusion of the at least one adrenergic agent, and that is substantially insoluble and inert in body fluids and compatible with body tissues; and wherein the first layer is disposed about the inner drug core so as to produce, when inserted or implanted a substantially constant rate of release of the adrenergic agent(s) from the device.

**2.** The device of claim 1, wherein said device is comprised of

(iii) one or more additional layers that are permeable to the passage of the adrenergic agent, and which are substantially insoluble and inert in body fluids and compatible with body tissues;

wherein the first and additional layers are disposed about the inner drug core so as to produce, when inserted or implanted, a substantially constant rate of release of the adrenergic agent from the device.

**3.** The device of claim 1 or 2, wherein the substantially impermeable layer has sufficient dimensional stability to be filled with an inner drug core without changing its tubular shape.

**4.** A sustained release drug delivery device adapted for insertion or implantation in or adjacent to the eye of a patient, the drug delivery device comprising:

(i) an inner drug core comprising at least one adrenergic agent and a polymer matrix material, wherein the adrenergic agent(s) is/are admixed in the polymer matrix material to inhibit or prevent decomposition of the adrenergic agent(s);
(ii) a layer on the surface of the drug core that is partially or substantially permeable to the passage of the at least one adrenergic agent, having one or more openings therein which aid diffusion of the at least one adrenergic agent, and that is substantially insoluble and inert in body fluids and compatible with body tissues;

and wherein the layer its either (i) disposed about the inner drug core so as to produce, when inserted or implanted, a substantially constant rate of release of the at least one adrenergic agent from the device or (ii) has sufficient dimensional stability to be filled with the inner drug core without changing its shape.

**5.** The device according to any one of claims 1 to 4 wherein the adrenergic agent is selected from brimodine, apraclonidine, bunazosin, timolol, betaxolol, levobetaxolol, levobunalol, carteolol, isoprenaline, fenoterol, metipranolol, clenbuterol, epinephrine, and dipiveirin.

**6.** The sustained release drug delivery device of claims 1 to 5, wherein the device is formed by co-extruding the inner drug core and the one or more layers.

**7.** The sustained release drug delivery device of claims 1 to 6, wherein the polymer matrix is bioerodible.

**8.** Use of an adrenergic agent for the preparation of a medicament,for the treatment and/or prevention of raised intraocular pressure, said medicament being incorporated into a sustained release device according to any one of claims 1 to 4, 6 and 7 whereby the device delivers the adrenergic agent to the ciliary body of the eye, wherein the adrenergic agent concentration in the ciliary body is maintained at a therapeutically effective concentration for a period of at least 30 days.

**9.** The use according to claim 8 wherein the adrenergic agent is selected from brimodine, apraclonidine, bunazosin, timolol, betaxolol, levobetaxolol, levobunalol, carteolol, isoprenaline, fenoterol, metipranolol, clenbuterol, epinephrine, and dipivefrin.

## Patentansprüche

**1.** Vorrichtung zur Arzneistoffabgabe mit verlängerter Freisetzung, angepasst zum Einfügen oder Implantieren in oder angrenzend an das Auge eines Patienten, wobei die Vorrichtung zur Arzneistoffabgabe umfasst:

(i) einen inneren Arzneistoffkern, umfassend mindestens einen adrenergen Wirkstoff und ein Polymer-Matrixmaterial, wobei der/die adrenerge(n) Wirkstoff(e) in dem Polymer-Matrixmaterial vermengt ist/sind, um die Zersetzung des/der adrenergen Wirkstoffs/Wirkstoffe zu hemmen oder zu verhindern;
(ii) eine erste Schicht auf der Oberfläche des inneren Arzneistoffkerns, welche im Wesentlichen undurchlässig für den Durchtritt des mindestens einen adrenergen Wirkstoffs ist, mit einer oder mehreren Öffnungen darin, welche die Diffusion des mindestens einen adrenergen Wirkstoffs ermöglichen, und welche im Wesentlichen unlöslich und inert in Körperflüssigkeiten und verträglich mit Körpergeweben ist; und

wobei die erste Schicht um den inneren Arzneistoffkern derart angeordnet ist, dass sie, wenn die Vorrichtung eingefügt oder implantiert ist, eine im Wesentlichen gleichbleibende Freisetzungsrate des/der adrenergen Wirkstoffs/Wirkstoffe aus der Vorrichtung bewirkt.

**2.** Vorrichtung gemäß Anspruch 1, wobei die Vorrichtung umfasst:

(iii) eine oder mehrere zusätzliche Schichten, welche für den Durchtritt des adrenergen Wirkstoffs durchlässig sind, und welche im Wesentlichen unlöslich und inert in Körperflüssigkeiten und verträglich mit Körpergeweben

sind;

wobei die erste und die zusätzlichen Schichten um den inneren Arzneistoffkern derart angeordnet sind, dass sie, wenn die Vorrichtung eingefügt oder implantiert ist, eine im Wesentlichen gleichbleibende Freisetzungsrate des adrenergen Wirkstoffs aus der Vorrichtung bewirken.

**3.** Vorrichtung gemäß Anspruch 1 oder 2, wobei die im Wesentlichen undurchlässige Schicht ausreichende Formbeständigkeit aufweist, um mit einem inneren Arzneistoffkern befüllt zu werden, ohne ihre röhrenförmige Form zu ändern.

**4.** Vorrichtung zur Arzneistoffabgabe mit verlängerter Freisetzung, angepasst zum Einfügen oder Implantieren in oder angrenzend an das Auge eines Patienten, wobei die Vorrichtung zur Arzneistoffabgabe umfasst:

(i) einen inneren Arzneistoffkern, umfassend mindestens einen adrenergen Wirkstoff und ein Polymer-Matrixmaterial, wobei der/die adrenerge(n) Wirkstoff(e) in dem Polymer-Matrixmaterial vermengt ist/sind, um die Zersetzung des/der adrenergen Wirkstoffs/Wirkstoffe zu hemmen oder zu verhindern;
(ii) eine Schicht auf der Oberfläche des Arzneistoffkerns, welche teilweise oder im Wesentlichen durchlässig für den Durchtritt des mindestens einen adrenergen Wirkstoffs ist, mit einer oder mehreren Öffnungen darin, welche die Diffusion des mindestens einen adrenergen Wirkstoffs unterstützen, und welche im Wesentlichen unlöslich und inert in Körperflüssigkeiten und verträglich mit Körpergeweben ist;

und wobei die Schicht entweder (i) um den inneren Arzneistoffkern derart angeordnet ist, dass sie, wenn die Vorrichtung eingefügt oder implantiert ist, eine im Wesentlichen gleichbleibende Freisetzungsrate des mindestens einen andrenergen Wirkstoffs aus- der Vorrichtung bewirkt, oder (ii) ausreichende Formbeständigkeit aufweist, um mit dem inneren Arzneistoffkern befüllt zu werden, ohne ihre Form zu ändern.

**5.** Vorrichtung gemäß einem der Ansprüche 1 bis 4, wobei der adrenerge Wirkstoff ausgewählt ist aus Brimodin, Apraclonidin, Bunazosin, Timolol, Betaxolol, Levobetaxolol, Levobunalol, Carteolol, Isoprenalin, Fenoterol, Metipranolol, Clenbuterol, Epinephrin und Dipivefrin.

**6.** Vorrichtung zur Arzneistoffabgabe mit verlängerter Freisetzung gemäß den Ansprüchen 1 bis 5, wobei die Vorrichtung durch Coextrudieren des inneren Arzneistoffkems und der einen oder mehreren Schichten gebildet ist.

**7.** Vorrichtung zur Arzneistoffabgabe mit verlängerter Freisetzung gemäß den Ansprüchen 1 bis 6, wobei die Polymer-Matrix bioerodierbar ist.

**8.** Verwendung eines adrenergen Wirkstoffs zur Herstellung eines Medikaments zur Behandlung und/oder Vorbeugung von erhöhtem Augeninnendruck, wobei das Medikament in eine Vorrichtung mit verlängerten Freisetzung gemäß einem der Ansprüche 1 bis 4, 6 und 7 inkorporiert, ist, wobei die Vorrichtung den adrenergen Wirkstoff in den Ziliarkörper des Auges abgibt, wobei die Konzentration des adrenergen Wirkstoffs im Ziliarkörper bei einer therapeutisch wirksamen Konzentration über einen Zeitraum von mindestens 30 Tagen aufrecht erhalten wird.

**9.** Verwendung gemäß Anspruch 8, wobei der adrenerge Wirkstoff ausgewählt ist aus Brimodin, Apraclonidin, Bunazosin, Timolol, Betaxolol, Levobetaxolol, Levobunalol, Carteolol, Isoprenalin, Fenoterol, Metipranolol, Clenbuterol, Epinephrin und Dipivefrin.

## Revendications

**1.** Dispositif d'administration de médicament à libération prolongée adapté à l'insertion ou l'implant dans ou proche de l'oeil d'un patient, le dispositif d'administration de médicament comprenant :

(i) un noyau de médicament interne comprenant au moins un agent adrénergique et une matrice polymère, dans lequel le ou les agents adrénergiques sont mélangés dans la matrice polymère pour inhiber ou empêcher la décomposition du ou des agents adrénergiques ;
(ii) une première couche sur la surface du noyau de médicament interne qui est substantiellement imperméable au passage du ou des agents adrénergiques, ayant une ou plusieurs ouvertures permettant la diffusion du ou des agents adrénergiques, et qui est substantiellement insoluble et inerte dans les fluides corporels et compatible

avec les tissus corporels ; et

dans lequel la première couche est placée autour du noyau de médicament interne de façon à produire, lorsqu'il est inséré ou implanté, une vitesse de libération substantiellement constante du ou des agents adrénergiques du dispositif.

**2.** Dispositif selon la revendication 1, dans lequel ledit dispositif comprend

(iii) une ou plusieurs couches additionnelles qui sont perméables au passage de l'agent adrénergique, et qui sont substantiellement insolubles et inertes dans les fluides corporels et compatibles avec les tissus corporels ;

dans lequel la première couche et les couches additionnelles sont placées autour du noyau de médicament interne de façon à produire, lorsqu'il est inséré ou implanté, une vitesse de libération substantiellement constante de l'agent adrénergique du dispositif.

**3.** Dispositif selon la revendication 1 ou 2, dans lequel la couche substantiellement imperméable a une stabilité dimensionnelle suffisante pour être remplie avec un noyau de médicament interne sans changement de sa forme tubulaire.

**4.** Dispositif d'administration de médicament à libération prolongée adapté à l'insertion ou l'implant dans ou proche de l'oeil d'un patient, le dispositif d'administration de médicament comprenant :

(i) un noyau de médicament interne comprenant au moins un agent adrénergique et une matrice polymère, dans lequel le ou les agents adrénergiques sont mélangés dans la matrice polymère pour inhiber ou empêcher la décomposition du ou des agents adrénergiques ;
(ii) une couche sur la surface du noyau de médicament qui est partiellement ou substantiellement perméable au passage d'au moins un agent adrénergique, ayant une ou plusieurs ouvertures favorisant la diffusion du ou des agents adrénergiques, et qui est substantiellement insoluble et inerte dans les fluides corporels et compatible avec les tissus corporels ; et

dans lequel la couche est soit (i) placée aux environs du noyau de médicament interne de façon à produire, lorsqu'il est inséré ou implanté, une vitesse de libération substantiellement constante du ou des agents adrénergiques du dispositif ou (ii) a une stabilité dimensionnelle suffisante pour être remplie avec le noyau de médicament interne sans changement de sa forme.

**5.** Dispositif selon l'une quelconque des revendications 1 à 4 dans lequel l'agent adrénergique est choisi parmi la brimodine, l'apraclonidine, la bunazosine, le timolol, le bétaxolol, le lévobétaxolol, le lévobunalol, le cartéolol, l'isoprénaline, le fénotérol, le métipranolol, le clenbutérol, l'épinéphrine, et la dipivéfrine.

**6.** Dispositif d'administration de médicament à libération prolongée selon l'une des revendications 1 à 5, dans lequel le dispositif est formé par co-extrusion du noyau de médicament interne et de la ou des couches.

**7.** Dispositif d'administration de médicament à libération prolongée selon l'une des revendications 1 à 6, dans lequel la matrice polymère est bioérodable.

**8.** Utilisation d'un agent adrénergique pour la préparation d'un médicament destiné au traitement et/ou à la prévention d'une augmentation de la pression intraoculaire, ledit médicament étant incorporé dans un dispositif à libération prolongée selon l'une quelconque des revendications 1 à 4, 6 et 7 le dispositif apportant l'agent adrénergique au corps ciliaire de l'oeil, dans laquelle la concentration en agent adrénergique dans le corps ciliaire est maintenue à une concentration thérapeutiquement efficace pendant une période d'au moins 30 jours.

**9.** Utilisation selon la revendication 8 dans laquelle l'agent adrénergique est choisi parmi la brimodine, l'apraclonidine, la bunazosine, le timolol, le bétaxolol, le lévobétaxolol, le lévobunalol, le cartéolol, l'isoprénaline, le fénotérol, le métipranolol, le clenbutérol, l'épinéphrine et la dipivéfrine.

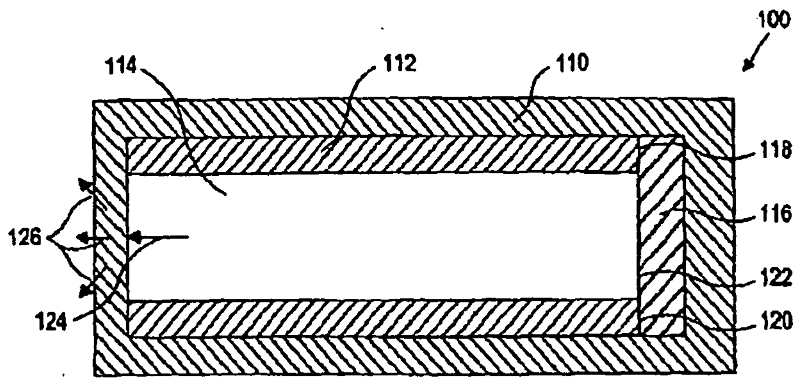


Fig. 1

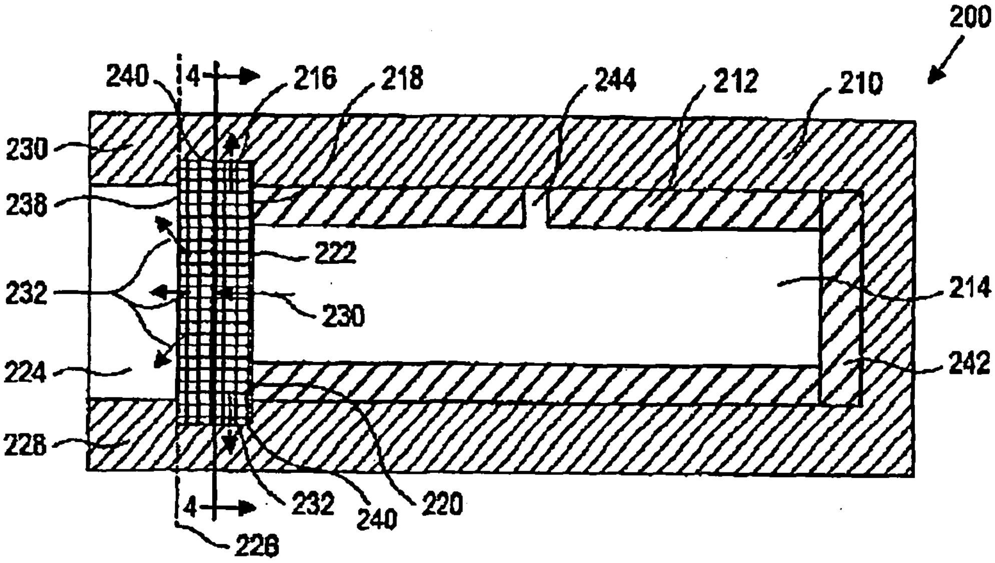
200
240
4
216
218
244
212
210
230
238
222
232
230
214
224
242
228
232
240
220
4
226

Fig. 2

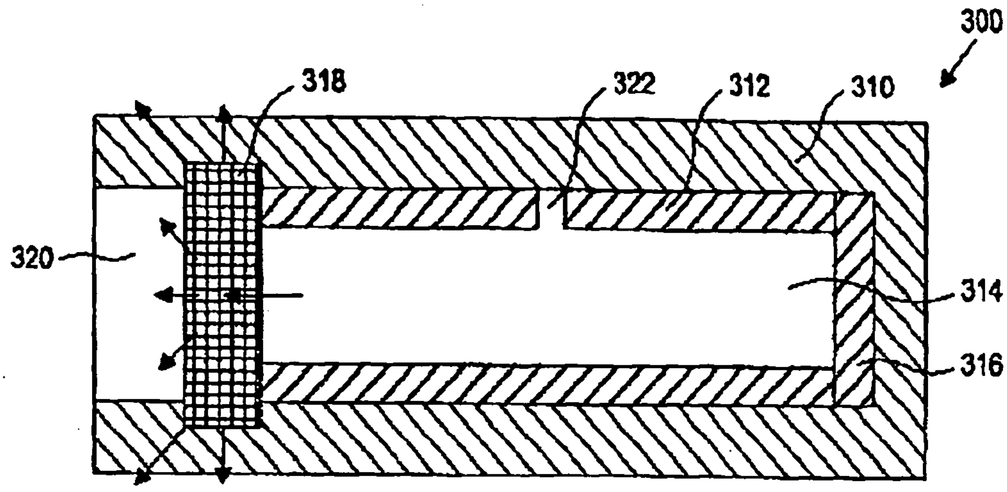


Fig. 3

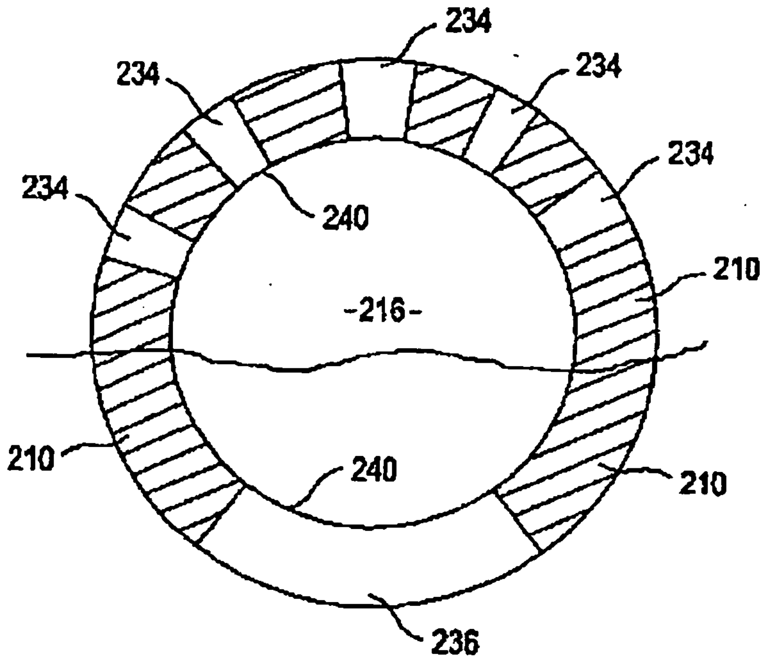


Fig. 4

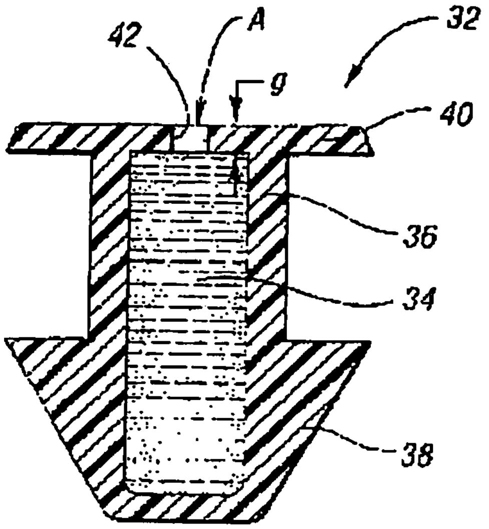


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6196993 B, Cohan and Diamond
- US 3416530 A
- US 3618604 A, Ness
- US 3626940 A, Zaffaroni
- US 3826258 A, Abraham
- US 3845201 A, Haddad and Loucas
- US 3845770 A, Theeuwes
- US 3962414 A, Michaels
- US 3993071 A, Higuchi
- US 4014335 A, Arnold
- US 4164559 A, Miyata
- US 5824072 A, Wong
- US 5476511 A, Gwon
- US 6416777 B, Yaacobi
- US 6027745 A, Nakada
- US 6368615 B, Guttag
- US 6217895 B
- US 6548078 B, Guo and Ashton
- US 5378475 A, Smith
- US 5902598 A, Chen and Ashton
- US 5773019 A
- US 6001386 A, Ashton and Pearson
- US 6375972 B, Guo and Ashton
- US 6051576 A
- US 428214 A
- US 483316 P
- US 4938763 A
- US 5077049 A
- US 5278202 A
- US 5324519 A
- US 5780044 A
- US 482677 P
- US 20020032298 A
- US 442499 P